# EUROPÄISCHE PATENTSCHRIFT

(11) **EP 0 712 830 B1**
(45) Veröffentlichungstag und Bekanntmachung des Hinweises auf die Patenterteilung: **12.08.1998**
(21) Anmeldenummer: 95117432.5
(22) Anmeldetag: 06.11.1995
(51) Int. Cl.: C07C 55/14, C07C 51/43

(54) **Verfahren zur Gewinnung von Adipinsäure**
Process for the recovery of adipic acid
Procédé de récupération d'acide adipique

(30) Priorität: 18.11.1994 DE 4441175
(43) Veröffentlichungstag der Anmeldung: 22.05.1996
(73) Patentinhaber: BAYER AG, 51368 Leverkusen (DE)
(72) Erfinder: Salzburg, Herbert, Dr., D-42799 Leichlingen (DE); Steinhoff, Georg, Dr., D-47800 Krefeld (DE); Gosch, Andreas, Dr., D-44789 Bochum (DE); Hufen, Gerd, D-47239 Duisburg (DE)

(56) Entgegenhaltungen:
- EP-A- 0 502 384
- US-A- 3 338 959

## Beschreibung

Die Erfindung betrifft ein Verfahren zur Gewinnung von Adipinsäure aus dem Nebenproduktausgrenzstrom (Mutterlauge), der bei der Oxidation technischer Gemische von Cyclohexanol/Cyclohexanon mittels Salpetersäure und Isolierung der Hauptmenge der gebildeten Adipinsäure durch Kristallisation anfällt, wobei gleichzeitig die in der genannten Mutterlauge verbleibenden Säuren als Gemisch ("Glutarsäure technisch") gewonnen werden.

Bei der Herstellung von Adipinsäure durch Oxidation von Cyclohexanol und/oder Cyclohexanon, insbesondere von technischen Gemischen als Cyclohexanol und Cyclohexanon mittels Salpetersäure fallen als Nebenprodukte Glutar- und Bernsteinsäure an. Diese sind in der Mutterlauge der Adipinsäurekristallisation angereichert. Ein Teil dieser Mutterlauge wird daher aus dem Prozeß ausgeschleust, um die Anreicherung der Nebenprodukte zu verhindern. Nach Abtrennung von Salpetersäure und Wasser aus dieser Mutterlauge kann das dann vorliegende Dicarbonsäuregemisch destillativ zu "Glutarsäure technisch" aufgearbeitet werden. Der dabei anfallende Destillationsrückstand enthält überwiegend Adipinsäure, Verkokungsprodukte sowie metallische Katalysatoren, wie z.B. Kupfer oder Vanadin.

Die in dem Dicarbonsäuregemisch enthaltene Adipinsäure (ca. 25 bis 45. Gew.-%) geht als Adipinsäure verloren, sie verschlechtert die Qualität der erhaltenen technischen Glutarsäure, beeinträchtigt somit ihre Verwendbarkeit im Gerbstoffbereich und erhöht die Rückstandsmenge bei der destillativen Glutarsäure-Aufarbeitung.

Zur Rückgewinnung von Adipinsäure wurden daher eine Reihe von Verfahren entwickelt. Gemäß US-PS 4 146 730 werden aus Glutar- und Bernsteinsäure einerseits und Harnstoff andererseits Addukte gebildet, die von der Adipinsäure abgetrennt werden können. US-PS 3 818 081 beschreibt die Umwandlung von Glutar- und Bernsteinsäure in die Imide und deren Abtrennung von der Adipinsäure. Gemäß EP-A-0 033 851 werden Glutar- und Bernsteinsäure durch Reaktion mit Alkylaminen in die entsprechenden Amide überführt, die ihrerseits von der Adipinsäure separiert werden können. US-PS 4 442 303 empfiehlt die Veresterung der Dicarbonsäuren und die Trennung der Säuren in Form ihrer Ester. US-PS 4 014 903 empfiehlt eine einfache Kühlkristallisation zur Rückgewinnung von Adipinsäure aus dem Nebenproduktausgrenzstrom. Die Effizienz eines solchen Verfahrens bleibt jedoch für ein technisches Verfahren unbefriedigend, weil die auf diese Weise gewinnbare Menge an Adipinsäure gering ist und insbesondere weil, wie auch in US-PS 4 254 283 bestätigt, viel Bernsteinsäure zusammen mit der Adipinsäure auskristallisiert, so daß ein weiterer Schritt zwecks Trennung dieser beiden Säuren erforderlich wird. Wie aus US-PS 4 254 283 ersichtlich, kann dies beispielsweise dergestalt erfolgen, daß man die Bernsteinsäure in Bernsteinsäureanhydrid überführt und dieses destillativ von der Adipinsäure abtrennt.

Es war daher die der Erfindung zugrundeliegende Aufgabe, ein neues Verfahren zur Rückgewinnung der in der genannten Mutterlauge vorliegenden Adipinsäure bereitzustellen, welches im Vergleich zu den genannten Verfahren des Standes der Technik auf einfache Weise durchführbar ist und andererseits zu Adipinsäure bzw. zu "technischer Glutarsäure" einer vergleichsweisen hohen Reinheit führt.

Diese Aufgabe konnte mit dem nachstehend näher beschriebenen erfindungsgemäßen Verfahren gelöst werden.

Gegenstand der Erfindung ist ein Verfahren zur Gewinnung von Adipinsäure aus bei der technischen Adipinsäureherstellung anfallender wäßriger, salpetersaurer Mutterlauge mit einem Gehalt von 2 bis 8 Gew.-% Bernsteinsäure, 4 bis 10 Gew.-% Glutarsäure und 2 bis 25 Gew.-% Adipinsäure bei einer- HNO₃-Konzentration, bezogen auf die vorliegende wäßrige Salpetersäure ohne Einbeziehung der genannten Säuren, von 40 bis 60 Gew.-%, dadurch gekennzeichnet, daß man
(i) die wäßrige Mutterlauge durch Eindampfen von Salpetersäure bis auf einen Restgehalt von maximal 2,5 Gew.-% HNO₃, bezogen auf das Gewicht des Eindampfrückstands, befreit,
(ii) das gemäß (i) erhaltene Konzentrat mit einer solchen Menge Wasser oder wäßriger Salpetersäure mit einem HNO₃-Gehalt von maximal 10 Gew.-% vermischt, die einem Gewichtsverhältnis von Feststoff zu Flüssigkeit von 1:2,1 bis 1:1,2 entspricht,
(iii) das gemäß (ii) zugesetzte Wasser bzw. die gemäß (ii) zugesetzte Salpetersäure während eines Zeitraums von mindestens 10 Minuten bei 20 bis 80°C auf das Konzentrat, gegebenenfalls unter fortlaufendem Durchmischen und gegebenenfalls unter zumindest teilweisem Lösen des Konzentrats, einwirken läßt und
(iv) die dann kristallin vorliegende bzw. die nach Abkühlen des gemäß Stufe (iii) erhaltenen Gemischs auf 10 bis 35°C auskristallisierende Adipinsäure von der flüssigen, Glutarsäure enthaltenden Phase abtrennt.

Bei dem beim erfindungsgemäßen Verfahren einzusetzenden Ausgangsmaterial handelt es sich um die oben bereits angesprochene Mutterlauge der technischen Adipinsäureproduktion durch Oxidation von Cyclohexanol und/oder Cyclohexanon, insbesondere von technischen Gemischen von Cyclohexanol und Cyclohexanon mittels Salpetersäure. Die Hauptmenge der bei dieser Umsetzung anfallenden Adipinsäure fällt in Form von leicht aus dem Reaktionsgemisch abtrennbarer fester Adipinsäure an. Die bei der Isolierung dieser festen Adipinsäure zurückbleibende, daß Ausgangsmaterial des erfindungsgemäßen Verfahrens bildende Mutterlauge enthält die bereits obengenannten Säuren in den obengenannten Mengen bei einer HNO₃-Konzentration der Mutterlauge, bezogen auf Wasser und HNO₃ ohne Einbeziehung sonstiger Bestandteile, von 40 bis 60 Gew.-%. Außerdem kann die beim erfindungsgemäßen Verfahren einzusetzende Mutterlauge geringe Konzentrationen an Schwermetallverbindungen, insbesondere Kupfer- oder Vanadiumverbindungen, wie sie bei der Oxidationsreaktion als Katalysatoren verwendet werden, enthalten. Die Gesamtkonzentration dieser Verunreinigungen, bezogen auf das Gesamtgewicht der Mutterlauge liegt im allgemeinen bei maximal 1 Gew.-%.

Außerdem können in der Mutterlauge sonstige, nicht identifizierte, zu Verfärbungen führende Verunreinigungen vorliegen.

In der Stufe (i) des erfindungsgemäßen Verfahrens wird die Mutterlauge zunächst durch Eindampfen von Salpetersäure und Wasser bis auf einen Restgehalt von maximal 2,5, vorzugsweise maximal 1,2 Gew.-% HNO₃ befreit.

In der Stufe (ii) des erfindungsgemäßen Verfahrens wird das so erhaltene Konzentrat mit Wasser oder wäßriger Salpetersäure mit einer HNO₃-Konzentration von bis zu 10 Gew.-%, vorzugsweise bis zu 3 Gew.-% vermischt, wobei die Menge des Wassers bzw. der verdünnten Salpetersäure so bemessen wird, daß ein Gewichtsverhältnis von Konzentrat-Feststoff zu Flüssigkeit von 1:2,1 bis 1:1,2, vorzugsweise 1:1,8 bis 1:1,3 vorliegt.

Während der Phase (iii) des erfindungsgemäßen Verfahrens läßt man das Wasser bzw. die verdünnte Salpetersäure auf das Konzentrat innerhalb des Temperaturbereichs von 20 bis 80°C, vorzugsweise 23 bis 70°C während eines Zeitraums von mindestens 10 Minuten, vorzugsweise mindestens 30 Minuten, einwirken. Dieses "Einwirken" wird vorzugsweise durch zumindest zeitweises Durchmischen, insbesondere durch zumindest zeitweises Rühren des Gemischs unterstützt.

Die Stufe (iv) des erfindungsgemäßen Verfahrens besteht in der Abtrennung von festen Kristallen aus dem nach der Phase (iii) vorliegenden Gemischs bei einer Temperatur von 10 bis 35, vorzugsweise 23 bis 30°C. Dies bedeutet, daß das gemäß Stufe (iii) erhaltene, bei höheren Temperaturen innerhalb der genannten Bereiche im allgemeinen eine Lösung darstellende Gemisch auf eine Temperatur innerhalb der letztgenannten Bereiche abgekühlt werden muß, falls die Behandlung gemäß Stufe (iii) bei einer Temperatur oberhalb 35°C durchgeführt worden war. Falls die Behandlung gemäß Stufe (iii) jedoch innerhalb der letztgenannten Temperaturbereiche erfolgte, ist eine Abkühlung nicht erforderlich. In einem solchen Falle bestünden die Stufen (iii) und (iv) in einer Durchmischung des Konzentrats mit dem Wasser bzw. der verdünnten Salpetersäure bei einer maximalen Temperatur von 35, vorzugsweise von 30°C und der anschließenden Isolierung der vorliegenden Säurekristalle. Die Isolierung der Säurekristalle kann beispielsweise durch Zentrifigieren oder durch Filtrieren erfolgen. Die Gesamtdauer der Wasser- bzw. Säure-Einwirkung gemäß Stufe (iii) und dem gegebenenfalls vorzunehmenden, vorzugsweise allmählich erfolgenden Abkühlen liegt im allgemeinen bei 10 Minuten bis 5 Stunden, vorzugsweise 30 bis 90 Minuten.

Die gemäß Stufe (iv) abgetrennte kristalline Säure stellt eine technische Adipinsäure mit einem Adipinsäuregehalt von mindestens 80 Gew.-% dar und enthält außerdem noch Restmengen an Glutar- und Bernsteinsäure. Dieses erste erfindungsgemäße Verfahrensprodukt kann beispielsweise in die Adipinsäureproduktion zurückgeführt werden und dort beispielsweise dem den Oxidationsreaktor verlassenden Reaktionsgemisch zugemischt werden.

Die-nach der Isolierung der technischen Adipinsäure gemäß Stufe (iv) vorliegende Mutterlauge enthält ein Säuregemisch, bestehend aus 40 bis 70 Gew.-% Glutarsäure, 10 bis 30 Gew.-% Adipinsäure und 20 bis 40 Gew.-% Bernsteinsäure. Diese "Glutarsäure technisch" kann durch Eindampfen dieser Mutterlauge (= fakultativ durchzuführende Stufen (v) des erfindungsgemäßen Verfahrens) im wesentlichen wasserfrei isoliert werden und stellt das zweite erfindungsgemäße Verfahrensprodukt dar. Diese "technische Glutarsäure" kann beispielsweise als Hilfsmittel in der Lederindustrie (Gerbung, Färbung) verwendet werden.

Bei der Durchführung des erfindungsgemäßen Verfahrens wird vorzugsweise darauf geachtet, daß möglichst farblose und von Schwermetallen befreite Verfahrensprodukte resultieren. Die Entfernung von Schwermetallspuren erfolgt vorzugsweise durch eine Ionenaustauscher-Behandlung, die im allgemeinen bei 10 bis 135°C durchgeführt wird. Hierfür geeignete Ionenaustauscher sind beispielsweise sauer eingestellte Kationenaustauscherharze auf Basis von Sulfonsäuregruppen aufweisenden Polystyrolen, wie sie beispielsweise unter der Bezeichnung ®Lewatit von der Bayer AG vertrieben werden.

Andere Verunreinigungen werden vorzugsweise durch eine Aktivkohle-Behandlung entfernt, die ebenfalls im allgemeinen bei 10 bis 135°C an einer beliebigen Stelle des Gesamtprozesses erfolgt.

So wäre es beispielsweise möglich das nach Beendigung der Stufe (i) vorliegende Konzentrat in der Schmelze bei 80 bis 135°C, vorzugsweise 90 bis 120°C den genannten Reinigungsoperationen zu unterziehen oder aber die wäßrige, in der Stufe (iv) anfallende Mutterlauge bei 10 bis 80°C, vorzugsweise 40 bis 65°C den genannten Reinigungsoperationen zu unterziehen.

In den nachfolgenden Beispielen beziehen sich alle Prozentangaben auf das Gewicht.

### Beispiele

In den nachfolgenden Beispielen 1 bis 7 und 12 bis 15 werden Mutterlauge-Konzentrate eingesetzt, die durch Eindampfen von Mutterlaugen aus der technischen Adipinsäureproduktion erhalten worden sind, und die einen HNO₃-Restgehalt von unter 0,7 Gew.-%, einen Bernsteinsäuregehalt von 20 bis 25 %, einen Glutarsäuregehalt von 33 bis 37 % sowie einen Adipinsäuregehalt von 36 bis 42 % aufweisen. Diese Konzentrate werden mit verdünnter Salpetersäure der nachstehend angegebenen Konzentration vermischt und unter Lösen auf 70°C erwärmt. Bei dieser Temperatur resultieren Lösungen mit dem nachstehend angegebenen Feststoffgehalt. Nach 5-minütigem Rühren bei 70°C wird während eines Zeitraums vonca. 90 Minuten auf 30°C abgekühlt und die kristallin vorliegende Adipinsäure abfiltriert. Das hierbei erhaltene Filtrat besteht im wesentlichen aus einer salpetersauren wäßrigen Lösung von "technischer Glutarsäure" der in nachstehender Tabelle angegebenen Zusammensetzung.

| Beispiel | HNO₃ % | FG¹⁾ % | AS²⁾ in FG % | AS in K³⁾ % | Ausbeute AS % | TGS⁴⁾ Zusammensetzung in % | | |
|---|---|---|---|---|---|---|---|---|
| | | | | | | AS⁵⁾ | GS⁶⁾ | BS⁷⁾ |
| 1 | 5 | 38 | 40 | 90,2 | 70,3 | 17,8 | 49,1 | 33,1 |
| 2 | 2 | 42,6 | 40 | 87,1 | 71,2 | 17,1 | 49,5 | 33,4 |
| 3 | 2 | 44 | 42 | 90,7 | 60,1 | 23,6 | 47,6 | 28,8 |
| 4 | 5 | 40 | 36 | 86,9 | 74,1 | 13,5 | 49,0 | 37,5 |
| 5 | 2 | 36 | 42 | 93,7 | 75,0 | 15,7 | 52,0 | 32,3 |
| 6 | 3 | 40 | 40 | 91,1 | 75,2 | 15,0 | 50,6 | 34,4 |
| 7 | 1,5 | 42 | 41 | 84,3 | 74,8 | 19,6 | 53,1 | 27,3 |

| | | | | | | | | |
|---|---|---|---|---|---|---|---|---|
| 1) Feststoffgehalt der Lösung (70°C) | | | | | | | | |
| 2) Adipinsäure | | | | | | | | |
| 3) Kristallisat | | | | | | | | |
| 4) "technische Glutarsäure" | | | | | | | | |
| 5) AS in TGS | | | | | | | | |
| 6) Glutarsäure in TGS | | | | | | | | |
| 7) Bernsteinsäure in TGS | | | | | | | | |

### Beispiel 8

Die Mutterlauge (4,9 g/l Cu, 350 mg/l V) der Kristallisation aus Beispiel 9 wird auf einer Kationenaustauschersäule (®Lewatit SP 112 der Bayer AG, 700 ml Bettvolumen) in der H-Form bei 50°C ausgetauscht. Austauschgeschwindigkeit ca. 450 ml/h. Einsatz 5.282 g, 36 % Feststoffgehalt. Das in einzelnen Fraktionen aufgefangene Eluat enthält 2,7 bis 4,3 ppm Cu und 3,4 ppm V.

### Beispiel 9

Das Eluat aus Beispiel 10 wird bei 37°C über eine mit 450 g Aktivkohle (2 bis 6 mm) gefüllte Säule gegeben und mit 250 ml Wasser nachgewaschen. Tropfgeschwindigkeit ca. 300 ml/h. Der Auslauf ist farblos. Durch Einengen erhält man 1900 g farblose "Glutarsäure technisch".

### Beispiel 10

Auf eine auf 110°C beheizte Säule mit 60 ml Füllung mit ®Lewatit SP 112 werden 20 g eines Gemisches aus 40 % Adipinsäure, 24 % Bernsteinsäure und 36 % Glutarsäure sowie 1 % Cu (als Cu(NO₃)₂) und 1,5 % V (als V₂O₅) (eingedampfte Mutterlauge aus dem technischen Adipinsäure-Herstellprozeß) gegeben, wobei dies Gemisch zuvor bei 90°C bis 100°C mit 3 g Wasser verrührt wurde.

Nach einem Vorlauf aus Wasser erhält man ca. 28,5 g Eluat, das bernsteinfarben ist und ca. 12 ppm Cu sowie 2 ppm V enthält.

### Beispiel 11

Auf eine auf 140°C geheizte Säule, die mit 49 g A-Kohle gefüllt ist, werden 500 g rohe, bernsteinfarbene, aufgeschmolzene "Glutarsäure technisch" gegeben, die durch Eindampfen des Eluates aus Beispiel 10 gewonnen worden ist. Das Produkt im Auslauf ist farblos.

### Beispiel 12-15

In den nachfolgenden Beispielen werden je 100 g Feststubstanz (24,9 % Bernsteinsäure, 35,0 % Glutarsäure, 40,0 % Adipinsäure) bei verschiedenen Temperaturen (T) je 180 min. mit Wasser verrührt, bei 30°C (bzw. 26°C in Beispiel 12) abgesaugt und die Zusammensetzung bestimmt.

| Nr. | T (°C) | Festkörpergehalt % | Kristallisat | | | T G S | | |
|---|---|---|---|---|---|---|---|---|
| | | | BS % | GS % | AS % | BS % | GS % | AS % |
| 12 | 26 | 43,5 | 5,4 | 7,4 | 87,1 | 34,0 | 47,9 | 18,1 |
| 13 | 30 | 40,0 | 4,4 | 6,0 | 89,6 | 33,1 | 46,7 | 20,2 |
| 14 | 32 | 40,0 | 4,2 | 6,4 | 89,1 | 33,3 | 46,6 | 20,1 |
| 15 | 80 | 40,0 | 5,8 | 7,7 | 86,5 | 33,6 | 47,4 | 19,0 |

## Patentansprüche

1. Verfahren zur Gewinnung von Adipinsäure aus bei der technischen Adipinsäureherstellung anfallender wäßriger, salpetersaurer Mutterlauge mit einem Gehalt von 2 bis 8 Gew.-% Bernsteinsäure, 4 bis 10 Gew.-% Glutarsäure und 2 bis 25 Gew.-% Adipinsäure bei einer HNO₃-Konzentration, bezogen auf die vorliegende wäßrige Salpetersäure ohne Einbeziehung der genannten Säuren, von 40 bis 60 Gew.-%, dadurch gekennzeichnet, daß man
(i) die wäßrige Mutterlauge durch Eindampfen von Salpetersäure bis auf einen Restgehalt von maximal 2,5 Gew.-% HNO₃, bezogen auf das Gewicht des Eindampfrückstands, befreit,
(ii) das gemäß (i) erhaltene Konzentrat mit einer solchen Menge Wasser oder wäßriger Salpetersäure mit einem HNO₃-Gehalt von maximal 10 Gew.-% vermischt, die einem Gewichtsverhältnis von Feststoff zu Flüssigkeit von 1:2,1 bis 1:1,2 entspricht,
(iii) das gemäß (ii) zugesetzte Wasser bzw. die gemäß (ii) zugesetzte Salpetersäure während eines Zeitraums von mindestens 10 Minuten bei 20 bis 80°C auf das Konzentrat, gegebenenfalls unter fortlaufendem Durchmischen und gegebenenfalls unter zumindest teilweisem Lösen des Konzentrats, einwirken läßt und
(iv) die dann kristallin vorliegende bzw. die nach Abkühlen des gemäß Stufe (iii) erhaltenen Gemisches auf 10 bis 35°C auskristallisierende Adipinsäure von der flüssigen, Glutarsäure enthaltenden Phase abtrennt.

2. Verfahren gemäß Anspruch 1, dadurch gekennzeichnet, daß man
(iii) das gemäß Stufe (ii) erhaltene Gemisch während eines Zeitraums von mindestens 30 Minuten bei 23 bis 70°C rührt und anschließend
(iv) die bei 23 bis 30°C vorliegende kristalline Adipinsäure bzw. die nach Abkühlen des Gemischs auf 30 bis 23°C auskristallisierende Adipinsäure von der Flüssigphase befreit.

3. Verfahren gemäß Anspruch 1 und 2, dadurch gekennzeichnet, daß man
(v) die gemäß Stufe (iv) erhaltene flüssige Phase unter Gewinnung eines Glutarsäure enthaltenden Säuregemischs durch Eindampfen von Wasser und Salpetersäure befreit.

4. Verfahren gemäß Anspruch 1 bis 3, dadurch gekennzeichnet, daß man aus dem System gegebenenfalls vorliegende Schwermetalle und gegebenenfalls vorliegende sonstige Verunreinigungen durch in beliebiger Reihenfolge und an beliebiger Stelle in Lösung oder in der Schmelze durchzuführende Ionenaustauscher- und/oder Aktivkohle-Behandlungen entfernt.

## Claims

1. Process for obtaining adipic acid from the aqueous, nitric-acid mother liquor arising from industrial adipic acid production having a content of 2 to 8 wt.% of succinic acid, 4 to 10 wt.% of glutaric acid and 2 to 25 wt.% of adipic acid at an HNO₃ concentration, relative to the aqueous nitric acid present without including the stated acids, of 40 to 60 wt.%,
characterised in that
(i) nitric acid is removed from the aqueous mother liquor by evaporation down to a residual content of at most 2.5 wt.% of HNO₃, relative to the weight of the evaporation residue,
(ii) the concentrate obtained according to (i) is mixed with such a quantity of water or aqueous nitric acid having an HNO₃ content of at most 10 wt.% as corresponds to a weight ratio of solid to liquid of 1:2.1 to 1:1.2,
(iii) the water added according to (ii) or the nitric acid added according to (ii) is allowed to act upon the concentrate for a period of at least 10 minutes at 20 to 80°C, optionally with continuous mixing and optionally with at least partial dissolution of the concentrate and
(iv) the adipic acid, which is then in crystalline form or crystallises out once the mixture obtained according to (iii) has cooled to 10 to 35°C, is separated from the liquid phase containing glutaric acid.

2. Process according to claim 1, characterised in that
(iii) the mixture obtained according to stage (ii) is stirred for a period of at least 30 minutes at 23 to 70°C and then
(iv) the crystalline adipic acid present at 23 to 30°C or the adipic acid which crystallises out once the mixture has cooled to 30 to 23°C is removed from the liquid phase.

3. Process according to claims 1 and 2, characterised in that
(v) water and nitric acid are removed by evaporation from the liquid phase obtained according to stage (iv) to obtain an acid mixture containing glutaric acid.

4. Process according to claims 1 to 3, characterised in that optionally present heavy metals and other optionally present contaminants are removed from the system by ion-exchanger and/or activated carbon treatments performed in solution or as a melt in any desired sequence and at any desired point.

## Revendications

1. Procédé pour récupérer l'acide adipique contenu dans les liqueurs-mères aqueuses nitriques formées à la fabrication industrielle de l'acide adipique qui contiennent de 2 à 8 % en poids d'acide succinique, 4 à 10 % en poids d'acide glutarique et 2 à 25 % en poids d'acide adipique à une concentration en HNO₃, rapportée à l'acide nitrique aqueux non tenu compte des acides organiques en question, de 40 à 60 % en poids, caractérisé en ce que
(i) on débarrasse les liqueurs-mères aqueuses par évaporation de l'acide nitrique jusqu'à une teneur résiduelle de 2,5 % en poids au maximum de HNO₃, par rapport au poids du résidu d'évaporation,
(ii) on mélange le concentré obtenu en (i) avec de l'eau ou de l'acide nitrique aqueux à une teneur maximale en HNO₃ de 10 % en poids, en quantité permettant de parvenir à un rapport en poids matières solides/liquide de 1 : 2,1 à 1 : 1,2,
(iii) on laisse agir l'eau ajoutée en (ii) ou l'acide nitrique ajouté en (ii) pendant une durée d'au moins 10 min à des températures de 20 à 80°C sur le concentré, le cas échéant en poursuivant le mélange et le cas échéant avec dissolution en partie au moins du concentré, et
(iv) on sépare l'acide adipique, qui a alors cristallisé ou qui cristallise après refroidissement à une température de 10 à 35°C du mélange obtenu au stade (iii), de la phase liquide contenant l'acide glutarique.

2. Procédé selon revendication 1, caractérisé en ce que
(iii) le mélange obtenu au stade (ii) est agité pendant une durée d'au moins 30 min à des températures de 23 à 70°C puis
(iv) l'acide adipique qui a cristallisé à des températures de 23 à 30°C ou qui cristallise après refroidissement du mélange à des températures de 30 à 23°C est débarrassé de la phase liquide.

3. Procédé selon revendications 1 et 2, caractérisé en ce que
(v) la phase liquide obtenue au stade (iv) est débarrassée par évaporation de l'eau et de l'acide nitrique, donnant un mélange d'acides qui contient de l'acide glutarique.

4. Procédé selon les revendications 1 à 3, caractérisé en ce que l'on élimine des opérations les métaux lourds éventuellement présents et d'autres impuretés éventuelles par des traitements sur échangeurs d'ions et/ou sur charbon actif exécutés dans un ordre quelconque et à un endroit quelconque sur des solutions ou des matières fondues.
